# EUROPEAN PATENT SPECIFICATION

(11) **EP 3 582 677 B1**
(45) Date of publication and mention of the grant of the patent: **13.10.2021**
(21) Application number: 18754934.0
(22) Date of filing: 16.02.2018
(51) Int. Cl.: A61B 3/10, G01B 9/02, G01N 21/47, G06T 7/00

(54) **SYSTEM AND A METHOD FOR DETECTING A MATERIAL IN REGION OF INTEREST**
SYSTEM UND VERFAHREN ZUM ERFASSEN EINES MATERIALS IN EINEM BEREICH VON INTERESSE
SYSTÈME ET PROCÉDÉ DE DÉTECTION D'UN MATÉRIAU DANS UNE RÉGION D'INTÉRÊT

(30) Priority: 17.02.2017 US 201762460384 P
(43) Date of publication of application: 25.12.2019
(73) Proprietor: The University Of Western Australia, Crawley, WA 6009 (AU)
(72) Inventor: CENSE, Abraham J., Nedlands, Western Australia 6009 (AU)
(74) Representative: Zimmermann, Tankred Klaus
(86) International application number: PCT/AU2018/050129
(87) International publication number: WO 2018/148805

(56) References cited:
- EP-A1- 2 896 355
- EP-A1- 2 896 355
- EP-A2- 3 054 420
- WO-A1-2010/085618
- WO-A1-2010/085618
- WO-A1-2010/122118
- WO-A1-2010/122118
- AU-A1- 2015 201 992
- AU-A1- 2015 201 992
- MICHAEL PIRCHER ET AL: "Polarization sensitive optical coherence tomography in the human eye", PROGRESS IN RETINAL AND EYE RESEARCH, vol. 30, no. 6, 26 June 2011 (2011-06-26), pages 431-451, XP028318331, ISSN: 1350-9462, DOI: 10.1016/J.PRETEYERES.2011.06.003 [retrieved on 2011-06-26]
- GOETZINGER ERICH ET AL: "Retinal pigment epithelium segmentation by polarization sensitive optical coherence tomography", OPTICS EXPRESS, OSA PUBLISHING, US, vol. 16, no. 21, 13 October 2008 (2008-10-13), pages 16410-16422, XP009192229, ISSN: 1094-4087
- PIRCHER, M. et al.: "Polarization sensitive optical coherence tomography in the human eye", Progress in Retinal and Eye Research, vol. 30, no. 6 November 2011 (2011-11), pages 431-451, XP028318331, Retrieved from the Internet: URL:https://www.ncbi.nlm.nih.gov/pmc/artic les/PMC3205186 [retrieved on 2018-03-15]
- GOTZINGER, E. et al.: "Retinal pigment epithelium segmentation by polarization sensitive optical coherence tomography", OPTICS EXPRESS, vol. 16 21 October 2008 (2008-10-21), pages 16410-16422, XP009192229, Retrieved from the Internet: URL:https://www.ncbi.nlm.nih.gov/pmc/artic les/PMC2976032 [retrieved on 2018-03-15]

## Description

### Field of the Invention

The present disclosure relates generally to a system and a method for detecting a material in region of interest and relates specifically, though not exclusively, to a system and method for detection of deposits within the region of interest and using polarization-sensitive optical coherence tomography ("PS OCT") .

### Background

Currently, there is no method to pinpoint deposits in the retinal pigment epithelium - Bruch' s membrane complex ("BM-RPE" complex) . Scanning laser polarimetry ("SLP") can be used to quantify polarization changes in the retina, in vivo, but since SLP only provides en face two-dimensional ("2D") image quality, changes cannot be linked to a specific depth.

EP 2 896 355 A1 describes a method of detecting and measuring of a geographic atrophy of the eye including imaging the eye at a depth greater than the retinal pigment epithelium (RPE) at a plurality of locations of the eye, for example, using optical coherence tomography (OCT); determining a ratio of the intensities of imaging signals of a retinal layer(s) with respect to the intensity of imaging signals of a sub-RPE layer(s) at each location; determining representative values based at least in part on the determined ratios; generating a map of the representative values; and identifying diseased areas from the map.

The present invention provides technological advancement.

### Summary of the Invention

In a first aspect of the present invention there is provided a method for detecting a material in a region of interest according to appended claim 1.

The method typically also comprises determining a threshold of the property. Determining the threshold of the property may comprise comparing depth images or depth image areas having the property, but which are not associated with the material with depth images or depth image areas having the property, but which are not associated with the material. In this case the depth images may be reference depth images of known regions of interest.

The step of processing the at least one depth image using the pre-determined threshold comprises comparing the at least one obtained depth image with (a library of) reference depth images to determine if the obtained depth image has a property below or above the pre-threshold.

The step of processing the at least one depth image using the pre-determined threshold may comprise filtering out or discriminating against depth image areas or depth images for which the property is above or below the threshold of the property.

The step of detecting the material in the region of interest may comprise analyzing the processed at least one depth image.

The step of providing at least one depth image may comprise using OCT imaging of the region of interest. The OCT imaging may be PS OCT.

The material may be a deposit.

The step of providing the at least one depth image may be performed in-vivo or ex-vivo. In one example the step of providing the at least one depth image is performed in-vivo and the region of interest is a region of interest of a subject, such as a patient. The region of interest may be a region within an eye of the subject, such as the retinal pigment epithelium of a subject's eye and the deposit may be a deposit within the in the retinal pigment epithelium (such as BM-RPE).

For example, the property may be a local intensity of an area or pixels within the at least one depth image. According to the invention the property is a retardance of electromagnetic waves as detectable with PS OCT (for example, by determining a change in polarization angle relative to a reference).

The OCT imaging may be PS OCT and may use a reference at an interface with a deposit at a location below or above the deposit. In one specific example the deposit is a deposit in the retinal pigment epithelium - Bruch's membrane complex of a subject's eye. An inner or outer segment of a photoreceptor layer may be used as a reference.

The method may be conducted to detect deposits located in a retinal pigment epithelium - Bruch's membrane complex of a subject's eye (such as in the BM-RPE) by detecting associated retardance. Further, the method may be conducted to detect retardance induced by a further deposit that is located below another deposit using at least one reference that is positioned above the at least one the other deposit.

In a second aspect of the present invention there is provided a system for detecting a material in region of interest, the system being configured to:
provide at least one depth image of the region of interest, the region of interest being below a surface area of the region of interest;
process the at least one depth image using a pre-determined threshold value of the property; and
detect the material in the region of interest by analyzing the processed at least one depth image.

The system typically is also configured to determine the threshold of the property of associated with the at least one depth image.

In a third aspect of the present invention there is provided a non-transitory computer-accessible medium having stored thereon computer-executable instructions for detecting a material in region of interest, the computer arrangement being configured to perform procedures comprising:
providing at least one depth image of the region of interest, the region of interest being below a surface area of the region of interest;
determining a threshold of a property of associated with the at least one depth image;
processing the at least one depth image using the determined threshold; and
detecting the material in the region of interest by analyzing the processed at least one depth image.

The computer-accessible medium may be configured to utilize inner and outer segments of a photoreceptor layer as a reference to collect the at least one retardance located below a retinal pigment epithelium (such as BM-RPE).

In a fourth aspect of the present invention there is provided a non-transitory computer-accessible medium having stored thereon computer-executable instructions for detecting at least one deposit in at least one subject, wherein, when a computer arrangement executes the instructions, the computer arrangement is configured to perform procedures comprising:
receiving information related to a reference information obtained from measurements in at least one further subject;
obtaining a threshold regarding at least one suspect data point in the information; and
detecting the at least one deposit in the at least one subject based on the threshold of at least one suspect data point.

The computer arrangement may be further configured to detect at least one retardance induced by a further deposit that is located below or above the at least one deposit using at least one reference that is positioned above or below the at least one deposit. The computer arrangement may also be configured to utilize inner and outer segments of a photoreceptor layer as a reference to collect the at least one retardance for a region below a retinal pigment epithelium (such as BM-RPE). Further, the computer arrangement may be configured to determine the at least one deposit using at least one light source having a central wavelength. The central wavelength may be about 840 nm. The computer arrangement may be further configured to detect the at least one deposit using a polarization having adaptive optics.

In a fifth aspect of the present invention there is provided a system for detecting at least one deposit in at least one subject, comprising:
using a specifically configured computer hardware arrangement configured to:
receive information related to a lookup table obtained from measurements in at least one further subject;
determine a threshold of at least one suspect data point in the information; and
detect the at least one deposit in the at least one subject based on the thresholded at least one suspect data point.

In a sixth aspect of the present invention there is provided a method for detecting at least one deposit in at least one subject, comprising:
receiving information related to a lookup table obtained from measurements in at least one further subject;
determine a threshold of at least one suspect data point in the information; and
using a specifically configured computer hardware arrangement, detecting the at least one deposit in the at least one subject based on the thresholded at least one suspect data point.

### Brief Description of the Drawings

Features and advantages of the present invention will become apparent from the following description of embodiments thereof, by way of example only, with reference to the accompanying drawings in which:
Figure 1 is a diagram of an exemplary spectral-domain Polarization-sensitive optical coherence tomography system according to an embodiment of the present invention;
Figures 2-3 are flow diagrams of a method of detecting a deposition according to an embodiment of the present invention;
Figure 4 is a histogram illustrating data obtained using a method in accordance with an embodiment of the present invention;
Figure 5 and 6 are images illustrating data obtained using a method in accordance with an embodiment of the present invention;
Figures 7A-7D are images produced using the system and method according to an embodiment of the present invention;
Figures 8A-8K are further exemplary images produced using the system and method according to an embodiment of the present invention; and

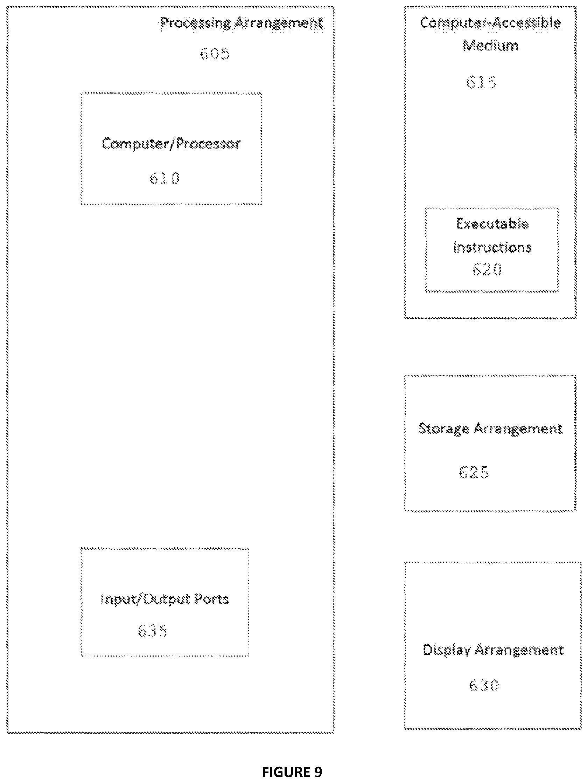

Figure 9 is an exemplary block diagram of a system in accordance with embodiments of the present invention.

### Detailed Description of Embodiments

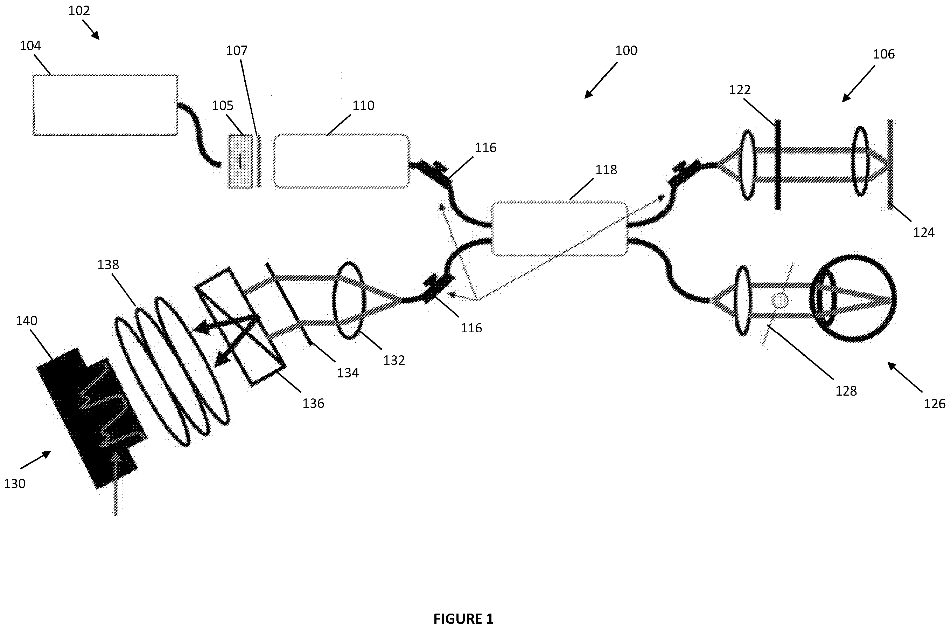

Figure 1 shows a diagram of a spectral-domain PS-OCT system 100 according to an embodiment of the present disclosure. The system 100 has a source arm 102 with a broadband light source 104. Further, the system 100 has a reference arm 106. In this example the broadband light source 104 emits light within a wavelength range centered around (840nm). In the source arm 102, light can be sent from the broadband light source 104 through an isolator 105 and two glass slides 107 for calibration. These glass slides can also be mounted in other arms of the interferometer, such as the reference arm 106. Information regarding the light can then proceed through a polarization modulator 110. The modulator 110 can apply two or more polarization states to the light, so that adjacent depth scans can be made with two orthogonal polarization states. Polarization controllers 116 are used to set the polarization state. The light is then split by a single-mode fiber coupler 118.

The reference arm 106 includes in this example a polarizer 122 and a mirror 124. The polarizer 122 is used in combination with the polarization controller 116 to ensure that the power returning to a detector can be the same for each polarization state, at each instant (e.g., even and odd depth scans, obtained with orthogonal input states).

The system 100 also has a sample arm 126 that includes in this example a raster scanning device 128 arranged to scan the retina of a subject.

The system 100 further has a detection arm 130. The detection arm 130 has in this example a polarization-sensitive high-speed spectrometer with a collimator 132, a transmission grating 134, a Wollaston prism 136, a focusing lens system 138 and line-scan camera 140. Detected spectra can be mapped to k-space, can be dispersion compensated, such that they can be Fourier transformed to depth without artifacts.

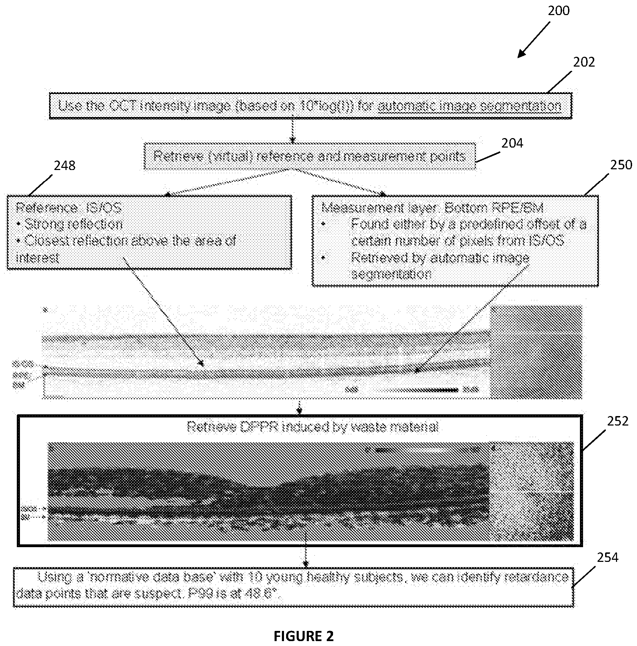

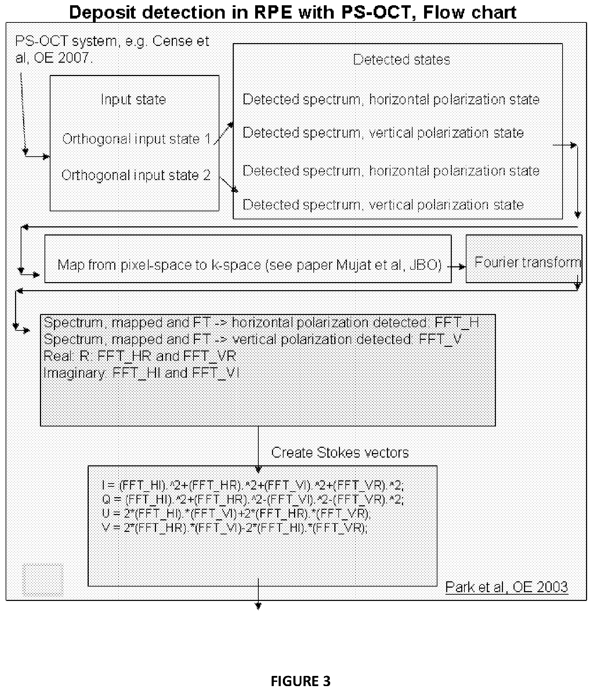

Figures 2 and 3 show flow diagrams of a method 200 of detecting a deposit in a subject's eye according to an embodiment of the present invention. The method 200 includes step 202, which uses OCT intensity images for automatic image segmentation of the inner and outer segments of the photoreceptor layer ("IS/OS") and the bottom of the RPE-BM complex of the subject's eye. Step 204 retrieves (virtual) reference and measurement points and step 250 performs a measurement for the bottom layer of the RPE-BM complex. The bottom edge of this complex can be detected in healthy subjects and subjects with minor drusen by offsetting the IS/OS by 66 µm. Step 248 used the IS/OS as a reference, as it usually provides high reflectivity, and is the closest reflector to the deposits in the RPE-BM complex. A double pass phase retardation ("DPPR") is calculated using Stokes-vector analysis with respect to the IS/OS as illustrated in flow chart shown in Figure 3. Step 252 of the method 200 retrieves DPPR and collects the retardation induced by deposits at the bottom of the RPE/BM complex. The measured retardation at this depth includes contributions of the photoreceptor outer tips, RPE cells, BM and deposits in Bruch's membrane.

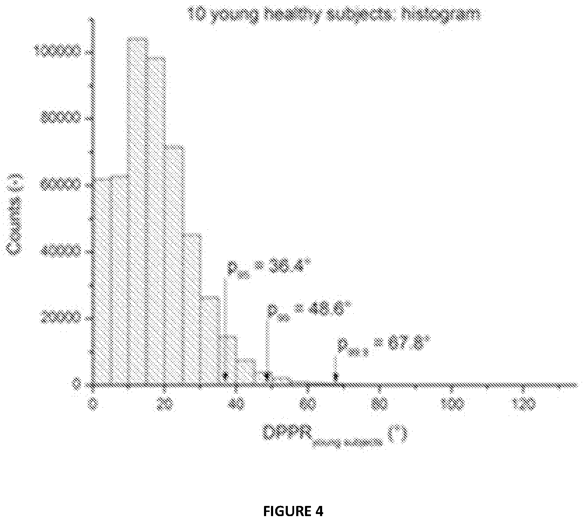

Step 254 of method 200 identifies retardance data points that are suspect using a "normal data base" with 10 young healthy suspects. Figure 4 is a histogram illustrating the data obtained from the 10 healthy subjects. This data was used to find a threshold at which data points become suspect. For example, the 99^{th} percentile (e.g., about 48.6°) was used to identify these points. At p99, the 99^{th} percentile, a DPPR cut-off is only found to be in 1% of all data points.

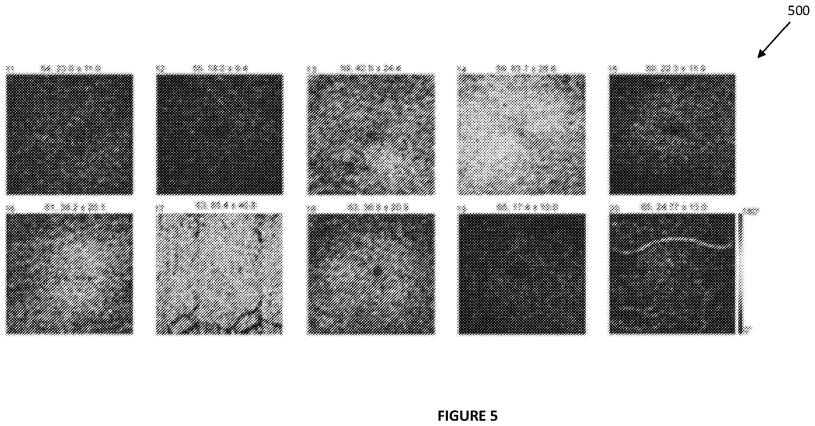

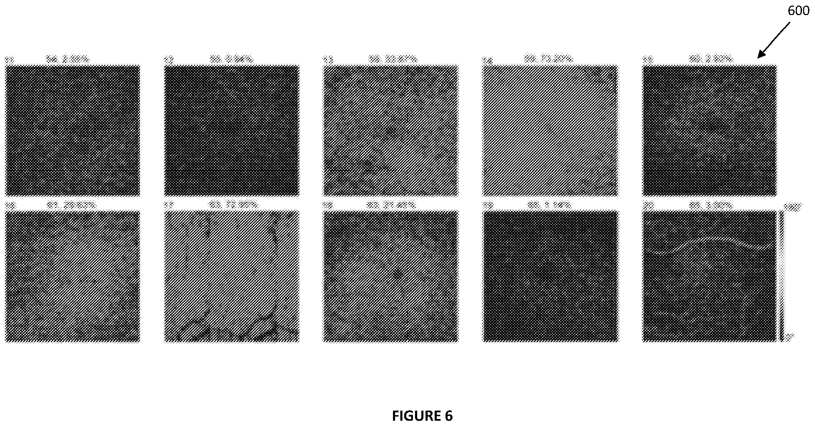

Figure 5 illustrates the data 500 obtained from 10 older subjects; two of these subjects, for example, subjects 13 and 14 (*see e.g.,* identifiers in the top left corner of each panel) were diagnosed as AMD patients; all other subjects did not have AMD. By applying a threshold at about 48.6°, suspect data points can be identified (see Figure 6, identified points or pixels shown in lighter colour). While there were three other subjects with significantly elevated DPPR values these subjects were not diagnosed with AMD, showing that the exemplary method can identify data points that can be pre-clinical. These subjects likely have thin deposits in their RPE/BM complex that have not matured into drusen.

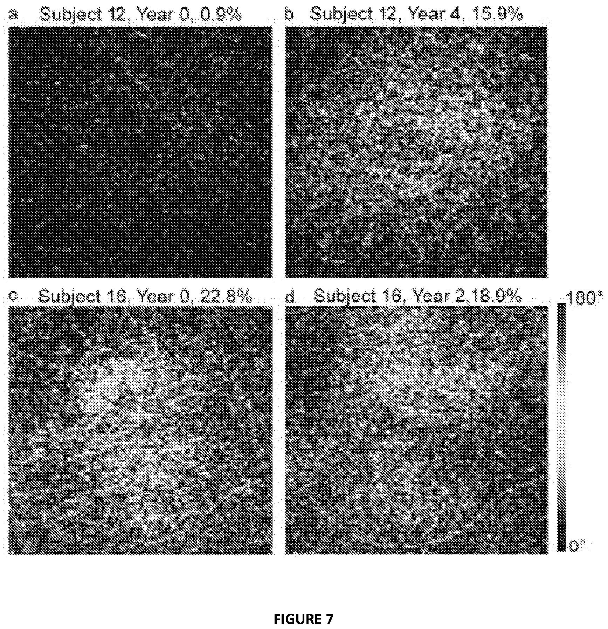

Figures 7A-7D show images produced using the system and method according to an embodiment of the present invention. The Figures show DPPR recorded below BM over about 15° by about 15° in a 59-year old subject in year 0 (e.g., Figure 3A), and after one year (e.g., Figure 3B). Figure 7A illustrates data obtained in a 55-year old in year 0, and Figure 7B shows the DPPR obtained after four years. The percentages above the images indicate how many data points were above the 99% cut-off of the 10 young subjects. The number of data points above the threshold increased with time, illustrating an accumulation of deposits.

Figures 7C and 7D show further exemplary images produced using the system and method according to an embodiment of the present invention. Figure 7C illustrates data obtained in a 61-year old in year 0, with 22.8% of data points above the threshold, indicating accumulation of deposits. Figure 7B shows the DPPR obtained after four years. Here, 19.8% of the data points were above the threshold.

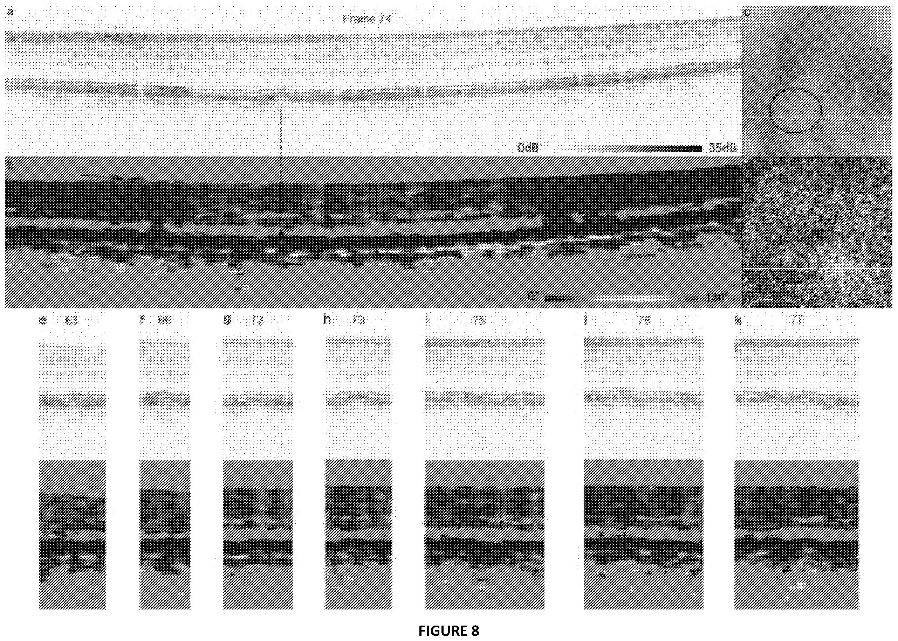

Figures 8A-8K show further images produced using the system and method according to an embodiment of the present invention. A druse can be found in the B-scan of subject 13 (e.g., Figure 8A) in frame 74 (e.g., the volume set has 100 frames). The segmented IS/OS and RPE/BM are not shown, to better visualize the structure of the drusen. The edges of this approximately 150-pm wide druse (e.g., Figures 8A and 8B) generate moderate DPPR of approximately 90°, while the center generates little DPPR at the depth of the RPE/BM border (e.g., Figure 8B). This can illustrate that the DPPR can be high when there can be little material between the RPE and BM, with decreasing DPPR with increased deposit thickness, due to hyalinization of the material. Similar observations were made for the other drusen in frames 63-77, within the area demarcated with the black ovals in Figures 8C and 8D, and also shown in Figures 8E-8K.

The use of PS-OCT for identifying deposits will now be summarized. Exemplary data was obtained with aPS-OCT system as described above with reference to Figures 1 to 3. The data was obtained on ten young healthy subjects and ten older subjects (e.g., 2-40). Two of these older subjects were diagnosed with AMD; all other subjects were graded as healthy without AMD.

The processing begins with *k*-space mapping and dispersion compensation. The real and imaginary parts of the acquired spectra can be converted into Stokes vectors. In this example each pixel in an image has four Stokes vectors I, Q, U and V. The retardance is calculated with respect to a reference.

The interface between the inner and outer segments of the photoreceptor layer (IS/OS) was used as a reference to avoid contamination of the retardance signal with contributions by the PS-OCT system itself, the cornea, retinal nerve fiber layer, and Henle fiber layer. A goal of the measurement was to quantify the retardance induced by the RPE-BM complex, which is located just below this reference.

The IS/OS reflect well, which provides a strong signal for reliable retardance calculation. The stronger reflections, the more reliable the DPPR calculation becomes. An objective is to detect polarization changes induced by deposits, which can occur in the RPE-BM complex, and it is beneficial to find a reference that can be close to these deposits, without being too close such that some spatial averaging can be applied. A reference that can be, for instance, at the top of the retina would not provide the same information, as birefringence contributions from the RNFL and the Henle fiber layer can contaminate the measurement that can be aimed at deposits in the RPE-BM complex. By using a reference at the top of the RNFL, the fast axis orientation of the RNFL can be used for retardance calculations, which can be different from the fast axis orientation of the tissue between the IS/OS and the RPE-BM complex, which can cause serious artifacts.

The IS/OS is beneficial for this purpose. The top of the RPE can also be used, but, as it is more closely spaced to the bottom of the RPE-BM complex, spatial averaging of Stokes vectors (which can be used to filter out speckle noise scrambling) can affect the retardance signal, as Stokes vectors at the top of the RPE can mix with Stokes vectors originating from the bottom of the RPE.

The IS/OS is in this example automatically segmented with image processing software.

Using the exemplary IS/OS as a reference, the retardance induced with respect to the IS/OS can be calculated. This is performed such that a color-coded image is generated, ranging from about 0° to about 180°, that is at about 0° at the IS/OS (see step 252 in Figure 2). In the direction towards the RNFL, and in the direction towards the choroid, an increase in DPPR is observed. The bottom of the RPE-BM complex can be of interest, as it can provide the retardance induced by deposits in the RPE-BM complex, the RPE and the outer tips of the photoreceptor layer.

The bottom of the RPE-BM complex is found by an offset of about 66 µm in the downward direction from the segmented IS/OS. Automatic segmentation also provides the location of the bottom of the RPE-BM complex. The DPPR at this location can be collected and displayed in *en face* images.

Data was collected on ten young healthy subjects, and made a histogram of the DPPR, which shows the distribution of the number of pixels as a function of DPPR magnitude. This Gaussian-shaped histogram shows the relationship between DPPR and percentiles: the 99^{th} percentile can be at a DPPR of 48.6°; the 99.9^{th} percentile can be at 67.8°. Depending on how strictly the data of patients can be thresholded, a certain cut-off to threshold data can be applied.

Data was also collected on 10 older subjects (aged 54-65). By applying the 99^{th} percentile threshold to these data sets, data points that can be suspect can be identified. The suspect data points is set to magenta (in this example).

These maps can be used to monitor the development of deposits. An increase of DPPR with time shows that the RPE-BM complex can be plagued by deposits, which can lead to AMD. However, at this stage, there may not be an indication that the patient suffers from the disease, which can first manifest itself as drusen.

The exemplary elevated retardance values (e.g. above the 99^{th} percentile threshold of 48.6°) are in this example linked to deposits. For example, data sets obtained from two subjects, taken four and two years apart is shown in Figures 7A-7D. When comparing the data collected in year 0 with data collected in year 2 and year 4, there can be an increase in DPPR, which can be mainly concentrated in areas that already had the highest DPPR in year 0. Areas with deposits can be likely to block the flow of nutrients and waste material, thereby collecting more deposits over time.

Additionally, Figures 8A-8K illustrate the DPPR signal below the drusen. The drusen are comprised of "waste material", and can look like mounds. The exemplary DPPR signal below the center of a druse is low, well below the 99^{th} percentile cut-off. At the edges of the druse, the DPPR signal is significantly higher. These images illustrate that when the collection of debris is still in an early stage, and the layer of debris is thin, such as at the edge of a druse, there is an elevated DPPR signal. In later stages, for example, when the subject already has AMD (the subject shown in Figures 8A-8K was confirmed with AMD), the DPPR signal drops to low values as the deposits is much thicker.

A distribution of the retardation that was recorded in the RPE-BM complex of ten young healthy subjects with spectral-domain PS-OCT at 840 nm is used to threshold data points that can be suspect, to identify areas with deposits in patients with age related macular degeneration or patients who are about to have age related macular degeneration.

As mentioned above, a reference for polarization-sensitive analysis can be located at the automatically segmented interface between the inner and outer segments of the photoreceptor layer (IS/OS); the retardance induced by deposits is recorded at or below the RPE-BM complex, which can either be found by an offset from the IS/OS, or by automatic image segmentation. The reference can alternatively also be located at the top of the RPE (towards the RNFL). A reference higher up in the retina, for example, at the top of the RNFL can also be used as a reference. While it can provide less accurate measurements, it can still provide some signal in the RPE-BM complex. This can benefit from a re-calibration of the data set illustrated in Figure 4, to determine the cut-off for an accurate threshold to detect deposits.

A spectral-domain system at any other central wavelength than 840 nm can be used (e.g. visible range, or near infra-red range near 1050 nm). Further, a swept-source PS-OCT system at any wavelength can be used. The exemplary system can be utilized with adaptive optics.

Figure 6 shows a block diagram of an embodiment of a system according to the present invention. Procedures can be performed by a processing arrangement and/or a computing arrangement 605. Such processing/computing arrangement 605 can be, for example entirely or a part of, or include, but not limited to, a computer/processor 610 that can include, for example one or more microprocessors, and use instructions stored on a computer-accessible medium (e.g., RAM, ROM, hard drive, or other storage device).

As shown in Figure 6, for example a computer-accessible medium 615 (e.g., as described herein above, a storage device such as a hard disk, floppy disk, memory stick, CD-ROM, RAM, ROM, etc., or a collection thereof) can be provided (e.g., in communication with the processing arrangement 605). The computer-accessible medium 615 can contain executable instructions 620 thereon. In addition or alternatively, a storage arrangement 625 can be provided separately from the computer-accessible medium 615, which can provide the instructions to the processing arrangement 605 so as to configure the processing arrangement to execute exemplary procedures, processes and methods, as described herein above.

Further, the exemplary processing arrangement 605 can be provided with or include an input/output arrangement 635, which can include, for example a wired network, a wireless network, the internet, an intranet, a data collection probe, a sensor, etc. As shown in Figure 6, the exemplary processing arrangement 605 is in communication with an exemplary display arrangement 630, which, can be a touch-screen configured for inputting information to the processing arrangement in addition to outputting information from the processing arrangement, for example. Further, the exemplary display 630 and/or a storage arrangement 625 can be used to display and/or store data in a user-accessible format and/or user-readable format.

The foregoing merely illustrates the principles of the disclosure. Various modifications and alterations to the described embodiments will be apparent to those skilled in the art in view of the teachings herein. It will thus be appreciated that those skilled in the art will be able to devise numerous systems, arrangements, and procedures which, although not explicitly shown or described herein, embody the principles of the disclosure and can be thus within the scope of the disclosure. Various different exemplary embodiments can be used together with one another, as well as interchangeably therewith, as should be understood by those having ordinary skill in the art. In addition, certain terms used in the present disclosure, including the specification, drawings and claims thereof, can be used synonymously in certain instances, including, but not limited to, for example, data and information. It should be understood that, while these words, and/or other words that can be synonymous to one another, can be used synonymously herein, that there can be instances when such words can be intended to not be used synonymously.

The following references are hereby provided for reference:
[1] Cense B, Wang Q, Lee S, Zhao L, Eisner AE, Hitzenberger CK, Miller DT . Henle fiber layer phase retardation measured with polarization-sensitive optical coherence tomography. Biomedical optics express. 2013 Nov 1 ; 4 (11) : 2296-306.
[2] Mujat M, Park BH, Cense B, Chen TC, de Boer JF .
   Autocalibration of spectral-domain optical coherence tomography spectrometers for in vivo quantitative retinal nerve fiber layer birefringence determination. Journal of biomedical optics. 2007 Jul; 12 (4) :041205.
[3] Chiu SJ, Li XT, Nicholas P, Toth CA,Izatt JA, Farsiu S. Automatic segmentation of seven retinal layers in SDOCT images congruent with expert manual segmentation. Optics express. 2010 Aug 30;18(18):19413-28.
[4] Park BH, Pierce MC, Cense B, de Boer JF. Real-time multifunctional optical coherence tomography. Optics express. 2003 Apr 7;11(7):782-93.
[5] Cense B, Chen TC, Park BH, Pierce MC, de Boer JF. In vivo depth-resolved birefringence measurements of the human retinal nerve fiber layer by polarization-sensitive optical coherence tomography. Optics letters. 2002 Sep 15;27(18):1610-2.
[6] Cense B, Chen TC, Park BH, Pierce MC, De Boer JF. Thickness and birefringence of healthy retinal nerve fiber layer tissue measured with polarization-sensitive optical coherence tomography. Investigative ophthalmology & visual science. 2004 Aug 1;45(8) :2606-12.
[7] Park BH, Pierce MC, Cense B, de Boer JF. Optic axis determination accuracy for fiber-based polarization-sensitive optical coherence tomography. Optics letters. 2005 Oct 1; 30(19) :2587-9.

## Claims

1. A computer implemented method for detecting a material in region of interest, comprising:
providing at least one depth image of the region of interest using polarization sensitive optical coherence tomography PS OCT imaging of the region of interest, the region of interest being below a surface area of the region of interest and showing a retardance of electromagnetic waves as directly or indirectly detectable with PS OCT;
processing the at least one depth image using a pre-determined threshold value of the retardance of electromagnetic waves, comprising comparing the at least one obtained depth image with at least one reference depth image to determine if the obtained depth image has a retardance of electromagnetic waves below or above the pre-determined threshold of the retardance of electromagnetic waves; and
detecting the material in the region of interest by analyzing the processed at least one depth image.

2. The method of claim 1 comprising determining the threshold of the retardance of electromagnetic waves.

3. The method of claim 2 wherein determining the threshold of the retardance of electromagnetic waves comprises comparing depth images or depth image areas having the retardance of electromagnetic waves and which are associated with the material with depth images or depth image areas having the retardance of electromagnetic waves, but which are not associated with the material.

4. The method of claim 3 wherein the depth images are reference depth images of a known region of interest.

5. The method of any one of the preceding claims wherein the step of processing the at least one depth image using the pre-determined threshold comprises filtering out or discriminating against depth image areas or depth images for which the retardance of electromagnetic waves is above or below the pre-determined threshold of the retardance of electromagnetic waves.

6. The method of any one of the preceding claims wherein the material is a deposit.

7. The method of any one of the preceding claims wherein the step of providing the at least one depth image is performed in-vivo and the region of interest is a region of interest of a subject and wherein the region of interest is a region within an eye of the subject.

8. The method of any one of the preceding claims wherein the property is associated with local intensity of an area or pixel within the at least one depth image.

9. The method of any one of the preceding claims wherein the PS OCT uses a reference at an interface with a deposit at a location below or above the deposit.

10. The method of claim 9 wherein the deposit is a deposit in the retinal pigment epithelium of a subject's eye.

11. The method of any one of the preceding claims wherein the method is conducted to detect deposits located in a region of a retinal pigment epithelium of a subject's eye by detecting associated retardance, such as the retinal pigment epithelium - Bruch' s membrane complex BM-RPE.

## Patentansprüche

1. Ein computerimplementiertes Verfahren zum Detektieren eines Materials in einem Bereich von Interesse, mit folgenden Schritten:
Bereitstellen zumindest eines Tiefenbildes des Bereiches von Interesse unter Verwendung von Polarisationssensitive-Optische-Kohärenztomographie, PS OCT,-Bildverarbeitung des Bereiches von Interesse, wobei der Bereich von Interesse unter einem Oberflächengebiet des Bereichs von Interesse liegt und eine Verzögerung elektromagnetischer Wellen als direkt oder indirekt mit PS-OCT detektierbar zeigt;
Verarbeiten des zumindest einen Tiefenbildes unter Verwendung eines vorbestimmten Schwellwerts der Verzögerung elektromagnetischer Wellen, wobei dies ein Vergleichen des zumindest einen erhaltenen Tiefenbildes mit zumindest einem Referenzbild aufweist, um zu bestimmen, ob das erhaltene Tiefenbild eine Verzögerung elektromagnetischer Wellen unterhalb oder oberhalb der vorbestimmten Schwelle der Verzögerung elektromagnetischer Wellen aufweist; und
Detektieren des Materials in dem Bereich von Interesse durch Analysieren des verarbeiteten zumindest einen Tiefenbildes.

2. Das Verfahren gemäß Anspruch 1, das ein Bestimmen der Schwelle der Verzögerung elektromagnetischer Wellen aufweist.

3. Das Verfahren gemäß Anspruch 2, bei dem das Bestimmen der Schwelle der Verzögerung elektromagnetischer Wellen ein Vergleichen von Tiefenbildern oder Tiefenbildgebiete, die die Verzögerung elektromagnetischer Wellen aufweisen und dem Material zugeordnet sind, mit Tiefenbildern oder Tiefenbildgebiete, die die Verzögerung elektromagnetischer Wellen aufweisen, jedoch nicht dem Material zugeordnet sind.

4. Das Verfahren gemäß Anspruch 3, bei dem die Tiefenbilder Referenztiefenbilder eines bekannten Bereiches von Interesse sind.

5. Das Verfahren gemäß einem der vorhergehenden Ansprüche, bei dem der Schritt des Verarbeitens des zumindest einen Tiefenbildes unter Verwendung der vorbestimmten Schwelle ein Herausfiltern oder Unterscheiden von Tiefenbildgebieten oder Tiefenbildern, für die die Verzögerung elektromagnetischer Wellen oberhalb oder unterhalb der vorbestimmten Schwelle der Verzögerung elektromagnetischer Wellen liegt, aufweist.

6. Das Verfahren gemäß einem der vorhergehenden Ansprüche, bei dem das Material eine Ablagerung ist.

7. Das Verfahren gemäß einem der vorhergehenden Ansprüche, bei dem der Schritt des Bereitstellens des zumindest einen Tiefenbildes in-vivo ausgeführt wird, und der Bereich von Interesse ein Bereich von Interesse einer Probanden ist und wobei der Bereich von Interesse ein Bereich innerhalb eines Auges des Probanden ist.

8. Das Verfahren gemäß einem der vorhergehenden Ansprüche, bei dem die Eigenschaft mit einer lokalen Intensität eines Bereichs oder Pixels in dem zumindest einen Tiefenbild in Verbindung steht.

9. Das Verfahren gemäß einem der vorhergehenden Ansprüche, bei dem die PS-OCT eine Referenz an einer Grenzfläche mit einer Ablagerung an einer Position unterhalb oder oberhalb der Ablagerung verwendet.

10. Das Verfahren gemäß Anspruch 9, bei dem die Ablagerung eine Ablagerung in dem retinalen Pigmentepithel eines Auges eines Probanden ist.

11. Das Verfahren gemäß einem der vorhergehenden Ansprüche, bei dem das Verfahren dazu ausgeführt wird, Ablagerungen zu detektieren, die sich in einem Bereich eines retinalen Pigmentepithels eines Auges eines Probanden befinden, durch Detektieren einer zugehörigen Verzögerung, etwa der Komplex retinales Pigmentepithel - Bruch-Membran BM-RPE.

## Revendications

1. Procédé mis en œuvre par ordinateur pour détecter un matériau dans une région d'intérêt, comprenant le fait de:
prévoir au moins une image de profondeur de la région d'intérêt à l'aide d'une imagerie tomographique par cohérence optique sensible à la polarisation PS OCT de la région d'intérêt, la région d'intérêt étant située au-dessous d'une zone de surface de la région d'intérêt et montrant un retard des ondes électromagnétiques comme pouvant être détecté directement ou indirectement par PS OCT;
traiter l'au moins une image de profondeur à l'aide d'une valeur de seuil prédéterminée du retard des ondes électromagnétiques, comprenant le fait de comparer l'au moins une image de profondeur obtenue avec au moins une image de profondeur de référence pour déterminer si l'image de profondeur obtenue présente un retard des ondes électromagnétiques au-dessous ou au-dessus du seuil prédéterminé du retard des ondes électromagnétiques; et
détecter le matériau dans la région d'intérêt en analysant l'au moins une image de profondeur traitée.

2. Procédé selon la revendication 1, comprenant le fait de déterminer le seuil de retard des ondes électromagnétiques.

3. Procédé selon la revendication 2, dans lequel la détermination du seuil du retard des ondes électromagnétiques comprend le fait de comparer les images de profondeur ou les zones d'image de profondeur présentant le retard des ondes électromagnétiques et qui sont associées au matériau avec des images de profondeur ou des zones d'image de profondeur présentant le retard des ondes électromagnétiques, mais qui ne sont pas associées au matériau.

4. Procédé selon la revendication 3, dans lequel les images de profondeur sont des images de profondeur de référence d'une région d'intérêt connue.

5. Procédé selon l'une quelconque des revendications précédentes, dans lequel l'étape consistant à traiter l'au moins une image de profondeur à l'aide du seuil prédéterminé comprend le fait de sortir par filtrage ou d'effectuer une discrimination des zones d'image de profondeur ou des images de profondeur pour lesquelles le retard des ondes électromagnétiques se situe au-dessus ou au-dessous du seuil prédéterminé de retard des ondes électromagnétiques.

6. Procédé selon l'une quelconque des revendications précédentes, dans lequel le matériau est un dépôt.

7. Procédé selon l'une quelconque des revendications précédentes, dans lequel l'étape consistant à prévoir l'au moins une image de profondeur est effectuée in-vivo et la région d'intérêt est une région d'intérêt d'un individu et dans lequel la région d'intérêt est une région située dans un œil de l'individu.

8. Procédé selon l'une quelconque des revendications précédentes, dans lequel la propriété est associée à l'intensité locale d'une zone ou d'un pixel dans l'au moins une image de profondeur.

9. Procédé selon l'une quelconque des revendications précédentes, dans lequel la PS OCT utilise une référence à une interface avec un dépôt à un emplacement au-dessous ou au-dessus du dépôt.

10. Procédé selon la revendication 9, dans lequel le dépôt est un dépôt dans l'épithélium pigmentaire rétinien de l'œil d'un individu.

11. Procédé selon l'une quelconque des revendications précédentes, dans lequel le procédé est réalisé pour détecter les dépôts situés dans une région d'un épithélium pigmentaire rétinien de l'œil d'un individu en détectant un retard associé, tel que complexe de l'épithélium pigmentaire rétinien - membrane de Bruch BM-RPE.
